# EUROPEAN PATENT APPLICATION

(11) **EP 2 878 340 A1**
(43) Date of publication of application: **03.06.2015**
(21) Application number: 13195202.0
(22) Date of filing: 29.11.2013
(51) Int. Cl.: A61Q 19/00, A61K 8/97, A61K 8/02, C12N 1/14

(54) **An abrasive ingredient for exfoliating cosmetic compositions**

(71) Applicant: Latvijas Universitate, 1586 Riga (LV)
(72) Inventor: Matjuskova, Natalja, LV1055 Riga (LV); Zala, Dzintra M., LV1029 Riga (LV); Azena, Elina, LV-1058 Riga (LV); Ramata-Stunda, Anna, LV-1011 Riga (LV); Silina, Karina, LV-1012 Riga (LV); Muiznieks, Indrikis, LV-1011 Riga (LV)
(74) Representative: Fortuna, Aleksandra

(57) **Abstract**

The present invention relates to use of grinded solid food manufacturing by-products for cultivation of basidiomycetes and subsequent application of cultivation products to produce exfoliating skin care products. There is proposed an abrasive ingredient for cosmetic compositions containing polysaccharides and other biologically active elements from fungal mycelium, where said abrasive ingredient is produced by liophylization of mycelium-covered grinded solid food waste particles, wherein the mycelium-covered grinded solid food waste particles are obtained by cultivation of basidiomycete mycelium in Malt Extract-based liquid cultivation medium with grinded solid food waste particles. According to one embodiment of the invention the mycelium is shiitake (*Lentinula edodes*) mycelium.

## Description

### Technical field

The present invention relates to production of abrasive components for inclusion in exfoliating skin care products. Abrasive component is produced biotechnologically and provides both skin exfoliation and care in one step.

### Prior art

Fungi and moulds contain compounds believed to have significant biologic activity, including triterpenes, proteins, lipids, cerebrosides, phenols, vitamins, fiber and amino acids. They also have been found to possess a high content of biologically active polysaccharides with demonstrated antitumor and immunostimulating properties. Edible basidiomycetes including *Lentinula edodes,* have shown health benefits both when taken orally or used topically (Takaku et al., 2001, Borchers et al., 2004)

Use of abrasives and peelings is beneficial in skin care as they help to remove dead skin corneocytes and helps to promote renewal of epidermis. Exfoliation of the skin can be undertaken by either chemical or mechanical means. Chemical exfoliation involves the process of applying to the skin a chemical which strips away the top layer of the skin. By contrast, mechanical exfoliation involves the removal of dead skin using coarse or granular materials. Minor stress induced by peelings and abrasives triggers microinflammatory responses and contributes not only to stimulation of epidermal regeneration, but also remodeling of extracellular matrix, dermal cell proliferation and communication between dermal and epidermal cells. Exfoliants help to loosen rough and dead skin through gentle abrasion of the skin. Natural exfoliants are derived from nuts (shell & meal), fruits (fibers, seeds), grains and other powdered or crystallized ingredients. They vary in their level of exfoliation depending on the mesh size and/or the hardness of the exfoliant. The following materials have been compounded in facial cleansers for the purpose of effectively removing foundation and make-up cosmetics: plant powders such as those obtained from hydrogenated jojoba oil and hydrogenated coconut oil, as well as the seeds and powders obtained from the apricot, almond, birch, walnut, peach, corn, sunflower, watermelon, etc powders of materials of animal origin such as powdered crab shell, powdered eggshell, etc., as well as those obtained from hydrogenated beef allow and hydrogenated lard; organic powders such as those obtained from polyethylene, nylon, polypropylene, such as those obtained from w polyvinyl chloride, polystyrene, and cellulose; and inorganic powders aluminum oxide, siiica, talc, and zirconium oxide (Cosmetics & Toiletries, Volume 101, July, 1986).

Examples of abrasive particles used in the formulation of scrub compositions are inorganic particles such as sea salt, silicates, powdered zeolite, coral powder, Kaolin, etc. The facial cleansers, which contain plant-based, animal-based, or inorganic powders as listed above, are hard, the geometry of particles is not spherical, but rather have acute angles, and as a consequence thereof, the these cleansers can damage the skin, cause spoilage and promote skin irritation when applied.

To overcome the drying and scratching problem, the skin scrub compositions are formulated using oils, humectants and moisturizers as carriers for the cosmetic scrub abrasive particles. For oily skins, however, aqueous scrub carrier is recommended thus alleviating, but not resolving to full extent the problem of scratching. Other solutions focus on the type and size of abrasive particles. Thus, some recopies of the composition of cosmetic scrubs make careful selection of abrasive materials to overcome the problem of wounding the skin during the use and the irritation afterwards.

In patent US 4,673,526, the use of water-insoluble polymeric materials that breaks at low shear force has been suggested as substitute to highly abrasive materials. However, to some people, they are still abrasive even in non-aqueous or oily media or emulsion. (WO2008056997 A2).

US patent 6,251,409 B1 provides a cosmetic composition with functional effect of cleansing and massage. Functional characteristics are achieved by incorporation of soft but expensive agarose particles in cosmetic formulations.

A challenge for cosmetic manufacturers is to combine skin cleansing properties and soothing properties in a single product. This is of a special interest for consumer groups with sensitive skin. As among other active cosmetic ingredients fungal polysaccharides, especially - glucans, and glycoproteins are widely used in skin moisturizers and products claiming regenerative characteristics, the approach combining abrasive particles with fungal polysaccharides in one product would be a solution of choice.

The use of cocoa bean shell and/or extract obtained after polar solvent extraction of cocoa shell as a component of the mushroom cultivation medium has been described by Japanese patent No 3804944, which claims the composition of solid cultivation media containing cocoa bean shells instead of saw dust from hardwood trees or sawdust-based cocoa bean extract supplemented solid media for growing of the fruiting bodies of various edible mushrooms, e.g. shiitake, enotake, maitake, champignons, etc.

### Disclosure of the invention

The invention relates to the production of abrasive cosmetic ingredient, which provides moisturizing and soothing effect along with the exfoliating capacity. There is proposed an abrasive ingredient for cosmetic compositions containing polysaccharides and other biologically active elements from fungal mycelium, where said abrasive ingredient is produced by liophylization of mycelium-covered grinded solid food waste particles, wherein the mycelium-covered grinded solid food waste particles are obtained by cultivation of basidiomycete mycelium in Malt Extract-based liquid cultivation medium with grinded solid food waste particles. According to one embodiment of the invention the mycelium is shiitake (*Lentinula edodes*) mycelium, obtainable by growing for 21-28 days at 24°C with continuous shaking on the rotator shaker at 140 rpm. The Malt Extract-based liquid cultivation medium preferably comprises cacao-bean shell particles having size in the range of 0.5 to 3.0 mm and concentration is in the range of 2.0-5.0% (w/v). The proposed abrasive ingredient can be used for skin care cosmetic compositions at concentrations 2.0-10.0 % (w/v), which will secure simultaneous removal of epidermal debris, skin moisturizing and releasing of the regenerative potential of skin cells.

Composition of cacao bean shells containing liquid medium. Cacao bean shells (CBS) are grinded with subsequent separation through the sieve with holes with definite diameter. The particles of definite sizes - from 0,5 mm to 3,0 mm are separated. Fractionated particles of CBS at 2,0 to 5,0% w/v concentration are added to the liquid malt extract (ME) based nutrient medium containing per litre: 17-30 g of dry malt extract, 0-3,0 g mycological peptone (pH 4,5-5,0) and sterilized in autoclave (121°C, 15 min) in 500 ml glass flasks containing 200 ml ME-CBS medium.

Growing of the fungal mycelium. ME-CBS medium was inoculated with shiitake mycelium covered ME agar disc, surface 1,0-1,5 cm² (strain DSM 3565 from the German Collection of Cell Cultures and Microorganisms) and placed on the rotary shaker (140 rpm, 24°C) for 3-4 weeks of cultivation. Afterwards the mycelium covered CBS particles were separated from the cultivation medium by filtration through the sieve and subjected to lyophilisation in Christ ALPHA 1-4 LD at main draying cycle at -35°C 0,22 mBar for 20 h; and final drying cycle at -55°C, 0.021 mBar, 0,5 h. The lyophilized material was stored at room temperature, isolated from atmospheric moisture.

### The use of mycelium covered lyophilized abrasive particles in cosmetic compositions

Cosmetic compositions of containing shiitake mycelium covered CBS were prepared as described in the examples given below.

### Example 1 - cleansing and regenerating facial scrub

Water (ingredient "a") and the mixture of ingredients (b) of the recipe provided below are heated to approximately 65°C until emulsifying waxes are completely melted; then (b) is added to (a) while vigorously stirring to form an emulsion. Stirring is continued until the formulation has cooled down to approximately 35 °C.

Lyophilized shiitake (*Lentinula edodes)* mycelium covered cacao (*Theobroma cacao*) bean shells are blended for 30 sec in coffee mill and mixed with ingredients listed in the part (c) of the recipe provided below.

Then the mixture (c) is added while stirring to rest of formulation.

All concentrations in the table below are shown v/v %, except for *L. edodes* covered *Theobroma cacao* bean shells and finely ground *Theobroma cacao* bean shells, where given concentrations are w/v %.

| | | |
|---|---|---|
| a) | Aqua | 63,7% |
| b) | Vitis vinifera (Grape) Seed Oil | 12,0% |
| | Caprylic/capric triglycerides | 8,0% |
| | Vegesilk (Hydrogenated Palm Kernel Glycerides, Hydrogenated Palm Glycerides) | 5,0% |
| | Olivem-100 (Cetearyl Olivate, Sorbitan Olivate) | 4,0% |
| | Silica | 1,0% |
| c) | *L. edodes* covered *Theobroma cacao* bean shells | 4,0% |
| | *Theobroma cacao* bean shells, finely ground | 1,0% |
| | Tocopherol acetate | 0,5% |
| | Potassium sorbate | 0,4% |
| | Gluconolactone, Sodium Benzoate. | 0,2% |
| | Naticide | 0,2% |

### Example 2 - exfoliating body scrub

Water (ingredient "a") and the mixture of ingredients (b) of the recipe provided below are heated to approximately 65°C until emulsifying waxes are completely melted; then (b) is added to (a) while vigorously stirring to form an emulsion. Stirring is continued until the formulation has cooled down to approximately 35 °C.

Lyophilized shiitake (*Lentinula edodes)* mycelium covered cacao (*Theobroma cacao*) bean shells are blended for 30 sec in coffee mill and mixed with ingredients listed in the part (c) of the recipe provided below.

Then the mixture (c) is added while stirring to rest of formulation.

All concentrations in the table below are shown v/v %, except for *L. edodes* covered *Theobroma cacao* bean shells and finely ground *Theobroma cacao* bean shells, where given concentrations are w/v %.

| | | |
|---|---|---|
| (a) | Aqua | 55,7% |
| (b) | Vitis vinifera (Grape) Seed Oil | 12,0% |
| | Cocos nucifera oil | 8,0% |
| | Vegesilk (Hydrogenated Palm Kernel Glycerides, Hydrogenated Palm Glycerides) | 5,0% |
| | Olivem-100 (Cetearyl Olivate, Sorbitan Olivate) | 4,0% |
| | Silica | 2,0% |
| (c) | *L. edodes* covered *Theobroma cacao* bean shells | 7,0% |
| | *Theobroma cacao* bean shells, finely ground | 5,0% |
| | Tocopherol acetate | 0,5% |
| | Potassium sorbate | 0,4% |
| | Gluconolactone, Sodium Benzoate. | 0,2% |
| | Naticide | 0,2% |

### References

1. Atsushi S, Atsumi N, Atsushi N, Susumu S. 2004. JP 3804944 (publication Nr. 2004-081123), Culture Medium for Culturing Mushroom and Method for Culturing the Same Mushroom.
2. Borchers AT, Keen CL, Gershwin EM. 2004. Mushrooms,Tumors, and Immunity: An Update. Experimental Biology and Medicine, 229:393-406.
3. Contamin JC, Zabotto A. 1987. US4673526 A, Anhydrous skin cleansing composition containing an oil phase, an emulsifying agent and particulate water soluble polymeric abrasive particles.
4. Hegyi E, Szathmary S, Grandics P. 2001. US6251409 B1, Use of particles in the composition of cosmetic products.
5. Hortaleza RB. 2008. WO2008056997 A2, Use of desiccated coconut as a scrubbing agent, askin scrub composition containing same, and method of making and using the scrub compositions.
6. Li W, Zhai ZH, Pang Q, Kong L, Zhou ZR. 2013. Influence of exfoliating facial cleanser on the bio-tribological properties of human skin. Wear 301: 353-361.
7. Takaku T, Kimura Y, Okuda H. 2001. Isolation of an Antitumor Compound from Agaricus blazei Murill and Its Mechanism of Action. Journal of Nutrition, 131: 1409-1413.

## Claims

1. An abrasive ingredient for cosmetic compositions containing polysaccharides and other biologically active elements from fungal mycelium, where said abrasive ingredient is produced by liophylization of mycelium-covered grinded solid food waste particles, wherein the mycelium-covered grinded solid food waste particles are obtained by cultivation of basidiomycete mycelium in Malt Extract-based liquid cultivation medium with grinded solid food waste particles.

2. The abrasive ingredient according to claim 1, wherein said mycelium is shiitake (*Lentinula edodes*) mycelium, obtainable by growing for 21-28 days at 24°C with continuous shaking on the rotator shaker at 140 rpm.

3. The abrasive ingredient according to claim 1 or 2, wherein the Malt Extract-based liquid cultivation medium further comprises cacao-bean shell particles.

4. The abrasive ingredient according to claim 3, wherein the cacao-bean shell particles' size is in the range of 0.5 to 3.0 mm.

5. The abrasive ingredient according to claim 3 or 4, wherein the cacao-bean shell particles' concentration is in the range of 2.0-5.0% (w/v).

6. Use of the abrasive ingredient according to any proceeding claims for skin care cosmetic compositions at concentrations 2.0-10.0 % (w/v).
